# EUROPEAN PATENT SPECIFICATION

(11) **EP 0 337 434 B1**
(45) Date of publication and mention of the grant of the patent: **20.10.1993**
(21) Application number: 89106530.2
(22) Date of filing: 12.04.1989
(51) Int. Cl.: C07C 45/54, C07C 67/475, C07C 69/16, C07C 45/46, C07C 67/42

(54) **Process for preparing diphenol monoesters substituted with aryl groups**
Verfahren zur Herstellung von mit Arylgruppen substituierten Diphenolmonoestern
Procédé pour fabriquer monoesters de diphénols substitués par groupes aryliques

(30) Priority: 12.04.1988 IT 2016888
(43) Date of publication of application: 18.10.1989
(73) Proprietor: HIMONT ITALIA S.r.l., I-20100 Milan (IT)
(72) Inventor: Borsotti, Giampiero, I-28100 Novara (IT); Foa', Marco, I-28100 Novara (IT)
(74) Representative: Zumstein, Fritz, Dr.

(56) References cited:
- EP-A- 0 060 419
- EP-A- 0 178 929

## Description

The present invention relates to a process for preparing diphenol monoesters substituted with aryl groups.

More particulary, the present invention relates to the preparation of diphenol monoesters substituted with aryl groups by starting with the corresponding mono-aryl-substituted phenols.

In organic chemistry, it is known to prepare phenols substituted with aryl groups, such as phenyl-hydroquinone, see, e.g. "Journal of Organic Chemistry" 1977, page 4071.

According to these techniques, phenyl-hydroquinone can be prepared from aniline, via a diazonium salt, by arylation whith benzoquinone, and subsequent reduction of the thus obtained phenyl-benzoquinone to the desired compound.

Such a process has practical drawbacks, in that it provides for the use of a potentially carcinogenic compound, such as aniline, and for the use of benzoquinone, which is a very costly reactant.

From Tetrahedron Letters 1983, page 5249, it is furthermore known to oxidate 2-phenyl-phenol to phenyl-benzoquinone with hydrogen peroxide in the presenca of ruthenium, with yields of 20%, and to reduce the thus obtained quinone, according to known techniques, to provide the corresponding hydroquinone.

According to all of these processes, the resulting diphenols have to be subjected to burdensome purification processes, in order to have the characteristics necessary for their intended end uses.

Such products, in fact, such as phenyl-hydroquinone, can be used as intermediates in organic syntheses or in the preparation of polyarylates and, in particular, of liquid-crystalline, thermotropic aromatic polyesters, such as disclosed in U.S. patent 4,159,365.

On the contrary, in organic chemistry, diphenol monoesters substituted with aryl groups, such as, e.g., phenyl- hydroquinone monoacetate, cannot be obtained from the corresponding diphenols by selective monoesterification. Diphenol monesters are considerably important in organic chemistry as intermediates for syntheses, or, as an alternative to the aryl-substituted diphenols known from the prior art, in the preparation of polyester resins.

In this case, they have the advantage, as compared to the corresponding phenols, in that they are not sensitive to the oxidating agents, and, therefore, are easier to handle and purify.

EP-A-178 929 discloses a process for preparing carboxylate esters of aromatic diols, such as phenyl-hydroquinone monoacetate, by subjecting an aromatic ketone containing a hydroxy, alkoxy or acyloxy radical on the aromatic ring to a Baeyer-Villiger oxydation using peracetic acid as oxidant under conditions such that the reaction mass contains no more than 0.1% of sulfuric acid based on the weight of the initially added pure peracetic acid.

The present invention provides a simple, inexpensive and high-yield process for preparing diphenol monoesters substituted with aryl groups, which comprises:
a) synthesizing, by means of a Friedel-Crafts reaction, a hydroxyketone from the corresponding mono-aryl-substituted phenol; and
b) oxydating said hydroxy-ketone to a diphenol monoester, by means of a Baeyer-Villiger reaction.

The Friedel-Crafts reaction proceeds according to the following reaction scheme: 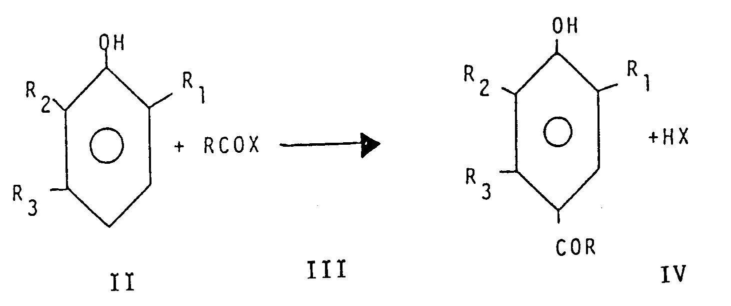
wherein:
- R: is an alkyl, a cycloalkyl, an aryl or alkylaryl radical containing from 1 to 12 carbon atoms, the aryl radical containing not less than 6 carbon atoms and the alkylaryl radical containing not less than 7 carbon atoms,
- R₁: is a simple, double or condensed (C₆-C₁₈)- aryl radical, optionally substitued with groups inert under the reaction conditions, preferably halogen and (C₁-C₅)alkyl radicals,
- R₂ and R₃: which may be the same or different are hydrogen, a C₁-C₁₅ alkyl radical, an aralkyl radical containing from 7 to 15 carbon atoms, or, when taken together, are the residue of an aromatic group containing from 6 to 18 carbon atoms, and
- X: is chlorine or bromine.

The Baeyer-Villiger reaction proceeds according to the following reaction scheme: 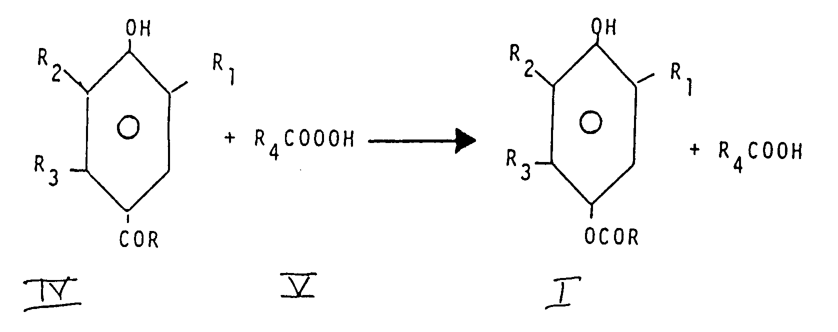
wherein
- R, R₁, R₂, R₃: have meanings disclosed above, and
- R₄: is hydrogen, or an alkyl radical, an aryl radical containing from 1 to 12 carbon atoms, or is selected from the groups consisting of 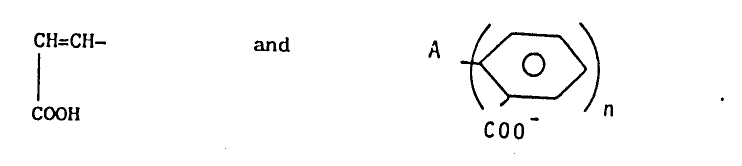
wherein
- A: is hydrogen, an alkali metal, or an alkali-earth metal, and n is 1 or 2. Preferred meanings of the above radicals are the following: R: methyl, ethyl, phenyl; R₁: phenyl, naphthyl; R₂ and R₃: hydrogen.

Any mono-aryl-substituted phenol of general formula (II) can be used in the process according to the present invention, even if ortho-phenyl-phenol is preferred, and among the acylating agents of general formula (III), acetyl chloride is preferred.

Such acylating agents are used in substantially equimolar ratios relative to the phenols used as the starting compounds.

The Friedel-Crafts reaction takes place at a temperature from 30 to 80°C, in the presence of a Lewis acid, and preferably in a solvent medium. Among the Lewis acids, aluminium chloride is preferred; and among the solvents compatible with the reaction conditions, nitrobenzene is preferred.

The Lewis acid is generally used according to molar ratios of at least 2:1, relative to the acylating agent. At the end of the reaction, the product obtained is recovered, after hydrolysis with water, by filtration, and washed with water and with suitable solvents, such as, e.g., aromatic solvents. The isolated ketone has a high degree of purity.

The Baeyer-Villiger reaction is carried out at a temperature comprised within the range of from -5 to 60°C. Any peracids falling within the scope of the general formula (V) can be used as oxidizing agents. Peracetic acid, perbenzoic acid, perphthalic acid, perlauric acid, perpropionic acid, permaleic acid and peroctanoic acid are preferred.

The peracids can be added or they can be formed in situ from the corresponding acid by reaction with aqueous solutions of hydrogen peroxide, preferably at a concentration of not less than 30% by weight.

The Baeyer-Villiger reaction is carried out in a solvent medium, and in the presence of an acidic catalyst.

Examples of solvents are acetic acid, propionic acid, ethanol and methylene chloride; the preferred catalysts are sulphuric acid, phosphoric acid, toluene-sulphonic acid, methane-sulphonic acid and trifluoro-acetic acid.

In the Baeyer-Villiger reaction, the amount of oxidating agent is generally in a stoichiometric ratio relative to the ketone. The catalyst is used in an amount from 10 to 60% by weight relative to the solvent medium.

A preferred product of the present invention is phenylhydroquinone mono-acetate, which is useful as an intermediate in organic syntheses, or as a comonomer in the synthesis of polyesters, particularly liquid-crystalline, thermotropic, aromatic polyesters.

The present invention is illustrated by the following non-limiting examples.

### Example 1

### Preparation of 2-phenyl-4-acetyl-phenol

To a 1 litre flask 170 g (1 mol) of ortho-phenyl-phenol, 300 ml of nitrobenzene and 1 g of aluminium chloride are charged; then, at the temperature of 25-30°C, 81 g of acetyl chloride (with a titre of 98%), and then 269 g of aluminium chloride are added dropwise, within one hour and within 30 minutes respectively: with the temperature being prevented from rising above 40-45°C. The reaction is allowed to proceed for 24 hours.

At the end of the reaction, a brown mass is obtained, which is poured into 600 ml of water and ice, until the whole mass is cooled down to 15°C, the precipitate is then filtered and is washed a plurality of times with toluene and water until a neutral pH value is obtained. It is then dried at 100°C.

183.3 g (yield of 86.1%) of a solid product, having a melting point of 175.5-176.5°C, corresponding to 2-phenyl-4-acetyl-phenol, is obtained.

TLC (thin-layer chromatography) and gas-chromatography of the product indicate that it is a single product.

### Example 2

To a 250-ml flask, 42.4 g of 2-phenyl-4-acetyl-phenol, prepared as disclosed in Example 1, and 100 ml of CH₃COOH are charged.

30 g of H₂SO₄ at 96% is added dropwise, with the temperature being kept at 15-20°C, the mass is then cooled down to 6-7°C, and 45 g of a solution of peracetic acid at 36% is added dropwise over 4 hours.

After an additional 2 hours, the disappearance of the starting product is observed. The suspension is then filtered. The precipitate is washed with a small amount of CH₃COOH (twice, with 10 ml each time), then with water until a neutral pH value is obtained. It is dried at 60°C under vacuum.
38.4 g of 2-phenyl-4-acetoxy-phenol with m.p. 154-155°C is obtained, with a yield of 84%.

TLC (thin-layer chromatography) and gas-chromatography of the product indicate that it is a single product.

### Example 3

Example 2 is repeated using, instead of the solution of peracetic acid available commercially, a peracetic acid prepared by mixing 37.5 g of CH₃COOH, 0.35 g of H₂SO₄ at 96% and 12.1 g of H₂O₂ at 70%, with the mixture being left standing for 24 hours.

38.4 g of product is obtained.

### Example 4

To a suspension of 21.2 g of 2-phenyl-4-acetyl-phenol in 50 ml of CH₃COOH, in a reaction vessel, a solution of peroctanoic acid, obtained by adding, at 20-25°C, 10 g of H₂O₂ at 50%, to a solution of 15 g of octanoic acid in 40 g of concentrated H₂SO₄, is added over 4 hours.

The temperature is gradually lowered from the initial temperature of 15°C to 6-7°C by the end of the addition. The mixture is stirred for additional 2 hours at 6-7°C, and is then filtered. The precipitate is washed twice with 10 ml of CH₃COOH, again with H₂O until a neutral pH is obtained. It is then dried at 60°C under vacuum. 18.8 g of 2-phenyl-4-acetoxy-phenol is obtained, with a yield of 82.4%.

### Example 5

To a suspension of 21.2 g of 2-phenyl-4-acetyl-phenol in 60 ml of propionic acid in a reaction vessel cooled at 5°C, a solution of perpropionic acid, prepared by adding, at 20-25°C, 8 g of H₂O₂at 50% to a solution of 8 g of propionic acid in 30 g of concentrated H₂SO₄, is added over a 4 hours.

At the end of the addition, the whole mass is stirred for an additional 2 hours, and is then filtered. The precipitate is washed twice with 10 ml of propionic acid, then with H₂O, then with a solution of NaHCO₃ at 5%, and again with water until a neutral pH is obtained. It is then dried under vacuum. 17.4 g of 2-phenyl-4-acetoxy-phenol is obtained, with a yield of 76%.

## Claims

1. A process for preparing diphenol monoester compounds of the formula 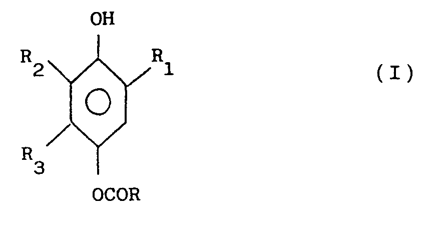 wherein:
R is an alkyl, a cycloalkyl, an aryl or an alkylaryl radical containing from 1 to 12 carbon atoms, the aryl radical containing not less than 6 carbon atoms and the alkylaryl radical containing not less than 7 carbon atoms,
R₁ is a simple, double or condensed (C₆-C₁₈)-aryl radical, optionally substituted with groups inert under the reaction conditions, preferably halogen and (C₁-C₅)-alkyl radicals,
R₂ and R₃, which may be the same or different, are hydrogen, a C₁-C₁₅ alkyl radical or an aralkyl radical containing from 7 to 15 carbon atoms, or, when taken together, are the residue of an aromatic group containing from 6 to 18 carbon atoms, caracterized in that in a first step an ortho-aryl-substituted phenol of formula (II) is treated with an acyl halide, in a solvent medium in the presence of a Friedel-Crafts catalyst, according to the following reaction scheme 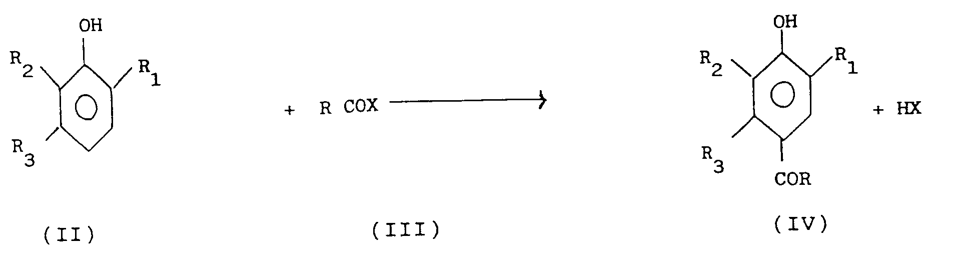 wherein R, R₁, R₂ and R₃ have the above meaning,
X is a chlorine or bromine
and in a second step the product (IV) is oxidized at a temperature from -5 to 60°C in the presence of an acidic catalyst in an amount from 10 to 60% by weight relative to the solvent medium, according to the following reaction scheme: 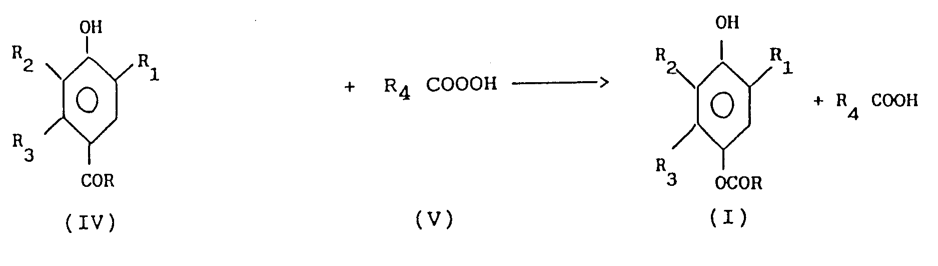 wherein:
R, R₁, R₂, R₃ have the above meaning and
R₄ is hydrogen or an alkyl radical, an aryl radical containing from 1 to 12 carbon atoms, or is selected from 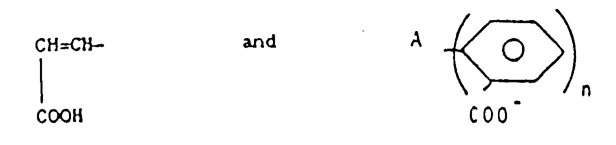 wherein A is hydrogen, an alkali metal, or an alkali-earth metal and n is 1 or 2.

2. The process of claim 1, wherein the mono-aryl-substituted phenol is ortho-phenyl-phenol.

3. The process of claims 1 or 2, wherein the acylating agent of general formula (III) is acetyl chloride.

4. The process of claims 1 to 3, wherein the Friedel-Crafts catalyst is aluminium chloride and the solvent is nitrobenzene.

5. The process of claims 1 to 4, wherein the oxidating agent of general formula (V) is selected from peracetic acid, perbenzoic acid, perphthalic acid, perlauric acid, perpropionic acid, permaleic acid and peroctanoic acid.

6. The process of claims 1 to 5, wherein the catalyst is selected from sulphuric acid, phosphoric acid, toluene-sulphonic acid, methane-sulphonic acid and trifluoro-acetic acid.

7. A process according to claim 1 in which the 2-phenyl-4-acetoxy-phenol is prepared by reacting ortho-phenyl-phenol in nitrobenzene solvent in the presence of aluminium chloride with acetyl chloride to give 2-phenyl-4-acetyl-phenol, which is subsequently oxidated at a temperature from -5°C to 60°C with an oxidating agent selected among peracetic acid, perbenzoic acid, perphthalic acid, perlauric acid, perpropionic acid, permaleic acid and peroctanoic acid, in the presence of sulfuric acid.

## Patentansprüche

1. Verfahren zur Herstellung von Diphenolmonoester-Verbindungen der Formel 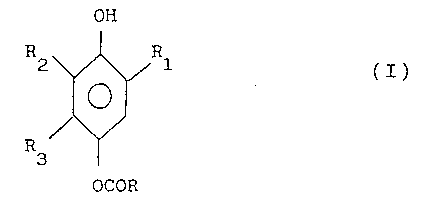 worin:
R eine Alkyl-, Cycloalkyl-, Aryl- oder Alkarylgruppe mit 1 bis 12 Kohlenstoffatomen ist, wobei die Arylgruppe nicht weniger als 6 Kohlenstoffatome aufweist und die Alkarylgruppe nicht weniger als 7 Kohlenstoffatome aufweist,
R₁ ein einfacher, doppelter oder kondensierter (C₆-C₁₈)-Arylrest, gegebenenfalls substituiert mit unter den Reaktionsbedingungen inerten Gruppen, vorzugsweise Halogen und (C₁-C₅)-Alkylresten, ist,
R₂ und R₃, die gleich oder verschieden sein können, Wasserstoff, einen C₁-C₁₅-Alkylrest oder einen Aralkylrest mit 7 bis 15 Kohlenstoffatomen bedeuten oder gemeinsam den Rest einer aromatischen Gruppe mit 6 bis 18 Kohlenstoffatomen bilden, dadurch gekennzeichnet, daß in einer ersten Stufe ein ortho-Aryl-substituiertes Phenol der Formel (II) mit einem Acylhalogenid in einem Lösungsmittelmedium in Gegenwart eines Friedel-Crafts-Katalysators nach dem folgenden Reaktionsschema 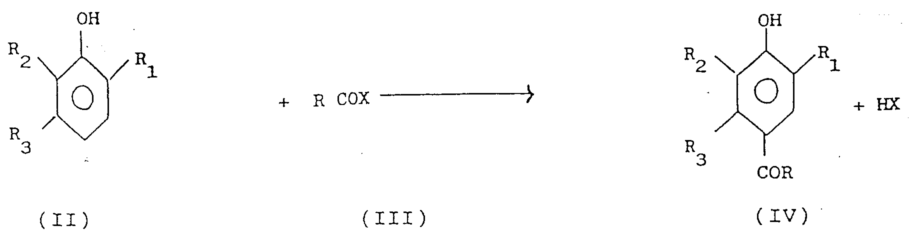 worin R, R₁, R₂ und R₃ die vorstehende Bedeutung besitzen,
X Chlor oder Brom ist,
behandelt wird,
und in einer zweiten Stufe das Produkt (IV) bei einer Temperatur von -5 bis 60°C in Gegenwart eines sauren Katalysators in einer Menge von 10 bis 60 Gew.-%, bezogen auf das Lösungsmittelmedium, nach dem folgenden Reaktionsschema 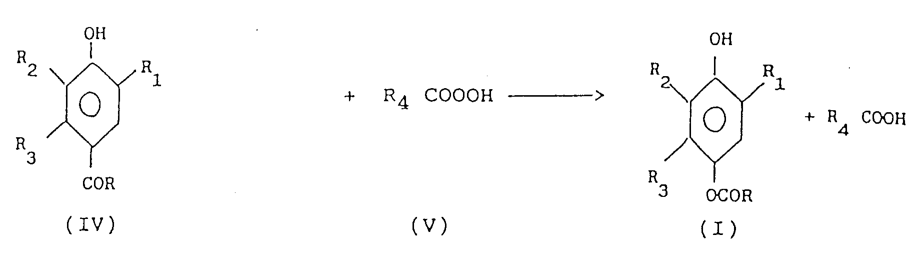 oxidiert wird, worin:
R, R₁, R₂, R₃ die vorstehende Bedeutung besitzen und
R₄ für Wasserstoff oder einen Alkylrest, einen Arylrest mit 1 bis 12 Kohlenstoffatomen steht oder ausgewählt ist unter 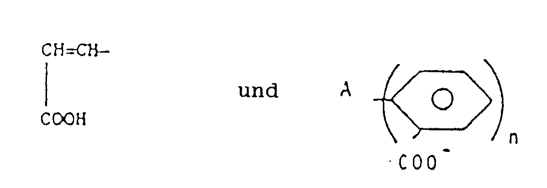 worin A Wasserstoff, ein Alkalimetall oder ein Erdalkalimetall bedeutet und n 1 oder 2 ist.

2. Verfahren gemäß Anspruch 1, worin das mono-Aryl-substituierte Phenol ortho-Phenylphenol ist.

3. Verfahren nach den Ansprüchen 1 oder 2, worin das Acylierungsmittel der allgemeinen Formel (III) Acetylchlorid ist.

4. Verfahren nach den Ansprüchen 1 bis 3, worin der Friedel-Crafts-Katalysator Aluminiumchlorid ist und das Lösungsmittel Nitrobenzol ist.

5. Verfahren nach den Ansprüchen 1 bis 4, worin das Oxidationsmittel der allgemeinen Formel (V) unter Peressigsäure, Perbenzoesäure, Perphthalsäure, Perlaurinsäure, Perpropionsäure, Permaleinsäure und Peroctansäure ausgewählt wird.

6. Verfahren nach den Ansprüchen 1 bis 5, worin der Katalysator unter Schwefelsäure, Phosphorsäure, Toluolsulfonsäure, Methansulfonsäure und Trifluoressigsäure ausgewählt wird.

7. Verfahren gemäß Anspruch 1, worin das 2-Phenyl-4-acetoxyphenol hergestellt wird durch Umsetzung von ortho-Phenylphenol in Nitrobenzollösungsmittel in Gegenwart von Aluminiumchlorid mit Acetylchlorid, um 2-Phenyl-4-acetylphenol zu ergeben, das anschließend bei einer Temperatur von -5 bis 60°C mit einem Oxidationsmittel, ausgewählt unter Peressigsäure, Perbenzoesäure, Perphthalsäure, Perlaurinsäure, Perpropionsäure, Permaleinsäure und Peroctansäure, in Gegenwart von Schwefelsäure oxidiert wird.

## Revendications

1. Un procédé de préparation de dérivés de monoester de diphénol de formule: 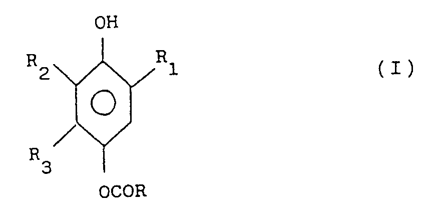 dans laquelle:
R représente un radical alkyle, cycloalkyle, aryle ou alkylaryle renfermant de 1 à 12 atomes de carbone, le radical aryle ne contenant pas moins de 6 atomes de carbone et le radical alkylaryle ne contenant pas moins de 7 atomes de carbone,
R₁ représente un radical aryle en C₆ à C₁₈ simple, double ou condensé, éventuellement substitué par des groupes inertes dans les conditions de la réaction, de préférence par un atome d'halogène et des radicaux alkyle en C₁ à C₅,
R₂ et R₃, qui peuvent être identiques ou différents, représentent un atome d'hydrogène, un radical alkyle en C₁ à C₁₅ ou un radical aralkyle contenant de 7 à 15 atomes de carbone ou, lorsqu'ils sont pris ensemble, ils représentent le résidu d'un groupe aromatique contenant de 6 à 18 atomes de carbone,
caractérisé en ce que, dans une première étape, un phénol substitué par un radical aryle en position ortho de formule (II) est traité par un halogénure d'acyle, dans un solvant, en présence d'un catalyseur de Friedel-Crafts selon le schéma réactionnel suivant: 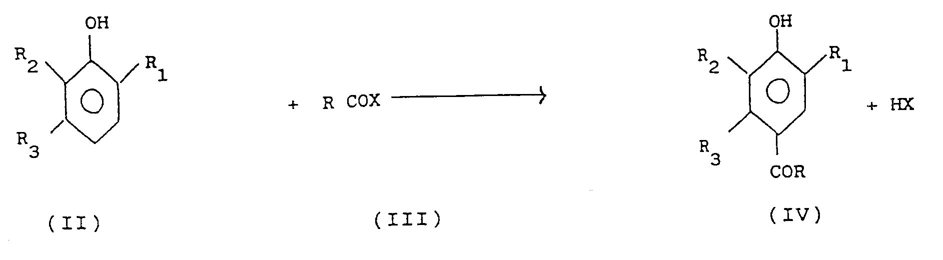 dans lequel:
R, R₁, R₂ et R₃ ont la signification indiquée ci-dessus,
X représente un atome de chlore ou de brome, et
dans une deuxième étape, le produit (IV) est oxydé à une température comprise entre -5°C à 60°C en présence d'un catalyseur acide en une quantité comprise entre 10 et 60% en poids par rapport au solvant selon le schéma réactionnel suivant: 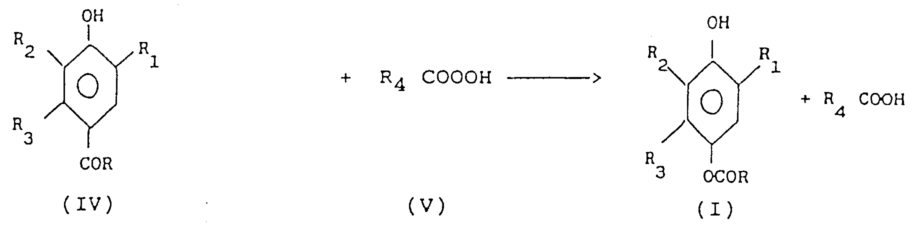 dans lequel:
R, R₁, R₂ et R₃ ont la signification indiquée ci-dessus, et
R₄ représente un atome d'hydrogène ou un radical alkyle, un radical aryle contenant de 1 à 12 atomes de carbone, ou il est choisi parmi: 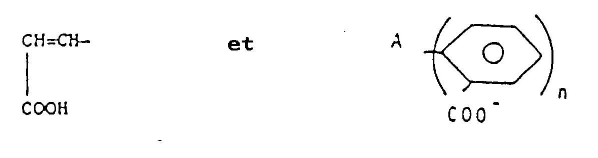 dans lequel:
A représente un atome d'hydrogène, un métal alcalin ou un métal alcalino-terreux, et
n est égal à 1 ou 2.

2. Procédé selon la revendication 1, caractérisé en ce que le phénol monosubstitué par un radical aryle est l'orthophénylphénol.

3. Procédé selon la revendication 1 ou 2, caractérisé en ce que l'agent d'acylation de formule générale (III) est le chlorure d'acétyle.

4. Procédé selon les revendications 1 à 3, caractérisé en ce que le catalyseur de Friedel-Crafts est le chlorure d'aluminium et le solvant est le nitrobenzène.

5. Procédé selon les revendications 1 à 4, caractérisé en ce que l'agent d'oxydation de formule générale (V) est choisi parmi l'acide peracétique, l'acide perbenzoïque, l'acide perphtalique, l'acide perlaurique, l'acide perpropionique, l'acide permaléique et l'acide peroctanoïque.

6. Procédé selon les revendications 1 à 5, caractérisé en ce que le catalyseur est choisi parmi: acide sulfurique, acide phosphorique, acide toluènesulfonique, acide méthanesulfonique et acide trifluoroacétique.

7. Procédé selon la revendication 1, caractérisé en ce que l'on prépare le 2-phényl-4-acétoxyphénol par réaction de l'orthophénylphénol dans du nitrobenzène comme solvant, en présence de chlorure d'aluminium et de chlorure d'acétyle pour obtenir le 2-phényl-4-acétoxyphénol qui est ultérieurement oxydé à une température de -5°C à 60°C par un agent d'oxydation choisi parmi: acide peracétique, acide perbenzoïque, acide perphtalique, acide perlaurique, acide perpropionique, acide permaléique et acide peroctanoïque en présence d'acide sulfurique.
